# EUROPÄISCHE PATENTANMELDUNG

(11) **EP 2 671 509 A1**
(43) Veröffentlichungstag der Anmeldung: **11.12.2013**
(21) Anmeldenummer: 13002908.5
(22) Anmeldetag: 05.06.2013
(51) Int. Cl.: A61B 5/113, A61M 16/00

(54) **Verfahren und Vorrichtung zur Atmungserkennung und/oder zur Atmungsunterstützung**

(30) Priorität: 06.06.2012 DE 102012011189
(71) Anmelder: Fritz Stephan GmbH, 56412 Gackenbach (DE)
(72) Erfinder: Braun, Wolfgang, 56428 Dernbach (DE); Höhne, Bernd, 56377 Nassau (DE); Schulze, Andreas, 82061 Neuried (DE)
(74) Vertreter: Bittner, Bernhard

(57) **Zusammenfassung**

Die Erfindung betrifft ein Verfahren zur Atmungserkennung und/oder zur Atmungsunterstützung eines Patienten mit einem Beatmungssystem zur synchronisierten Beatmung des Patienten, bei welchem mittels wenigstens einem mit dem Patienten wechselwirkenden Sensorelements eine Eigenatmung des Patienten ermittelt wird, und bei welchem das Beatmungssystem durch mittels des wenigstens einen mit dem Patienten wechselwirkenden Sensorelements erzeugte Signale gesteuert wird, wobei das wenigstens eine mit dem Patienten wechselwirkende Sensorelement und/oder die hierdurch erzeugten Signale während der Ermittlung der Eigenatmung kalibriert werden, indem die erzeugten Signale durch Bilden wenigstens eines arithmetischen Mittelwertes in Bezug auf zuvor erzeugte Signale rechnerisch korrigiert werden.

## Beschreibung

Die Erfindung betrifft ein Verfahren zur Atmungserkennung und/oder zur Atmungsunterstützung eines Patienten mit einem Beatmungssystem zur synchronisierten Beatmung des Patienten, bei welchem mittels wenigstens einem mit dem Patienten wechselwirkenden Sensorelements eine Eigenatmung des Patienten ermittelt wird, und bei welchem das Beatmungssystem durch mittels des wenigstens einen mit dem Patienten wechselwirkenden Sensorelements erzeugte Signale gesteuert wird.

Die Erfindung betrifft des Weiteren eine Vorrichtung zur Atmungserkennung und/oder zur Atmungsunterstützung eines Patienten mit einem Beatmungssystem zur synchronisierten Beatmung des Patienten, mit Mitteln zur Ermittlung einer Eigenatmung des Patienten und mit Mitteln zur synchronisierten Steuerung des Beatmungssystems in Abhängigkeit von einer ermittelten Eigenatmung, bei welcher die Mittel zur Ermittlung der Eigenatmung wenigstens ein mit dem Patienten wechselwirkendes Sensorelement umfassen.

Insofern liegt die Erfindung auf dem Gebiet der Atmungserkennung und im Speziellen auf dem Gebiet der Steuerung einer Atmungsunterstützung bei der Beatmung eines Patienten. Aus dem Stand der Technik sind insbesondere verschiedene Verfahren zur Erkennung der Atmung eines Patienten und zur Abgleichung eines entsprechenden Respirators eines Beatmungssystems mit der Atmung des Patienten bekannt. Bei solchen bekannten Systemen erfolgt ein Abgleich über ein am Körper des Patienten angebrachtes Kapselelement bzw. Sensorelement, mit welchem die Atemtätigkeit des Patienten ermittelt werden kann. Dieser Abgleich wird speziell dazu genutzt, einen Patienten bei einer ausbleibenden Eigenatmung zwangszubeatmen, etwa im Zusammenhang mit einer Apnoebeatmung. Somit wird der Abgleich auch zur Steuerung des diesbezüglichen Respirators verwendet. Derartige herkömmliche Verfahren und Beatmungssysteme erweisen sich allerdings aufgrund ihrer oftmaligen Fehleranfälligkeit als nachteilig.

Es ist Aufgabe der Erfindung, gattungsgemäße Verfahren und entsprechende Beatmungssysteme weiterzuentwickeln, um eine Überwachung einer Atemtätigkeit und eine gegebenenfalls erforderliche Unterstützung einer Eigenatmung eines Patienten sicherer und genauer zu gestalten.

Die Aufgabe der Erfindung wird durch ein Verfahren zur Atmungserkennung und/oder zur Atmungsunterstützung eines Patienten mit einem Beatmungssystem zur synchronisierten Beatmung des Patienten gelöst, bei welchem mittels wenigstens einem mit dem Patienten wechselwirkenden Sensorelements eine Eigenatmung des Patienten ermittelt wird, und bei welchem das Beatmungssystem durch mittels des wenigstens einen mit dem Patienten wechselwirkenden Sensorelements erzeugte Signale gesteuert wird, wobei das wenigstens eine mit dem Patienten wechselwirkende Sensorelement und/oder die hierdurch erzeugten Signale während der Ermittlung der Eigenatmung kalibriert werden, indem die erzeugten Signale rechnerisch korrigiert werden.

Dabei ist es möglich, dass die erzeugten Signale kalibriert durch Anwenden wenigstens einer mathematischen Operation und insbesondere durch Bilden wenigstens eines (insbesondere aber nicht ausschließlich arithmetischen) Mittelwertes in Bezug auf zuvor erzeugte Signale korrigiert werden. Es könnten jedoch auch andere Operationen angewandt werden etwa die Bildung geometrischer Mittelwerte, Integrationen oder dergleichen. Auch könnten Operationen wie Extremwertberechnungen durchgeführt werden.

Eine entsprechende Signalaufbereitung ist insofern derart gestaltet, dass erfindungsgemäß fortlaufend eine Kalibrierung des Sensorelements bzw. diesbezüglich erzeugter Signale erfolgt. Diese Kalibrierung ist idealerweise derart angelegt, dass ein Ruhesignal immer wieder auf einen vorfestlegbaren Ursprungswert zurück findet. Hierzu wird erfindungsgemäß wenigstens ein arithmetischer Mittelwert verwendet, der aus zuvor erzeugten Signalen ermittelt wird.

Das erfindungsgemäße Verfahren ermöglicht somit eine äußerst zuverlässige direkte Überwachung der Atemtätigkeit des Patienten und/oder eine sehr präzise direkte Steuerung der Beatmung des Patienten.

Jedenfalls ist mittels des vorliegenden Verfahrens eine deutliche Vereinfachung, Präzisierung und damit Optimierung einer Beatmungssteuerung erzielt.

Die Verbesserungen hinsichtlich des Verfahrens beziehen sich sowohl auf den Umgang mit den erzeugten Signalen als auch auf die Art und Weise, wie die erzeugten Signale das Beatmungssystem und insbesondere einen diesbezüglichen Respirator beeinflussen, wie nachstehend noch erläutert ist.

Der Begriff- "erzeugte Signale" bezieht sich auf die von dem Sensorelement in Abhängigkeit von einer durch die Atemtätigkeit eines Patienten ausgelösten Signalerzeugung. Insofern handelt es sich bei den vorliegend durch das wenigstens eine Sensorelement erzeugten Signalen insbesondere um Atemtätigkeitssignale, welche durch die Atemtätigkeit des Patienten ausgelöst sind.

Darüber hinaus wird die Aufgabe der Erfindung von einer Vorrichtung zur Atmungserkennung und/oder zur Atmungsunterstützung eines Patienten mit einem Beatmungssystem zur synchronisierten Beatmung des Patienten, mit Mitteln zur Ermittlung einer Eigenatmung des Patienten und mit Mitteln zur synchronisierten Steuerung des Beatmungssystems in Abhängigkeit von einer ermittelten Eigenatmung gelöst, bei welcher die Mittel zur Ermittlung der Eigenatmung wenigstens ein mit dem Patienten wechselwirkendes Sensorelement umfassen, wobei sich die Vorrichtung durch Kalibrierungsmittel zur Kalibrierung des wenigstens einen mit dem Patienten wechselwirkenden Sensorelements auszeichnet, mittels welchen das wenigstens eine mit dem Patienten wechselwirkende Sensorelement und/oder der hierdurch erzeugten Signale während der Ermittlung der Eigenatmung kalibriert werden.

Mit der erfindungsgemäßen Vorrichtung kann insbesondere das vorliegende Verfahren vorteilhaft durchgeführt werden.

Um eine rechnerische Kalibrierung einfach vornehmen zu können, ist es vorteilhaft, wenn die Vorrichtung eine Recheneinrichtung umfasst, mittels welcher eine entsprechende Berechnung eines derartigen Mittelwertes erfolgen kann.

Diese Recheneinrichtung kann konstruktiv sehr einfach bereitgestellt werden, wenn sie in einer entsprechend ausgestalteten Elektronikeinrichtung der Vorrichtung bzw. eines Respirators implementiert ist.

Ferner kann sowohl das erfindungsgemäße Verfahren als auch die erfindungsgemäße Vorrichtung insbesondere auch bei einer nicht-invasiven Beatmung von Früh- und/oder Neugeborenen zur direkten Überwachung der Atemtätigkeit eines diesbezüglichen Patienten verwendet werden.

Durch die Kalibrierungsmittel ist baulich besonders einfach sichergestellt, dass die erzeugten Signale stets äußerst präzise zur lebenswichtigen Steuerung des Beatmungssystems bereitgestellt werden.

In Zusammenhang mit dieser apparativen Verbesserung ist somit auch die Verbesserung der Signalaufbereitung zu sehen.

Es versteht sich, dass eine wirkungsvolle Kalibrierung auf unterschiedlicher Weise erfolgen kann. Eine nicht nur konstruktiv einfache Kalibrierung kann erzielt werden, wenn die Kalibrierungsmittel eine Einrichtung zum Betätigen eines Ventilelements zur Belüftung und/oder Entlüftung des Beatmungssystems umfasst.

Insofern ist es vorteilhaft, wenn die Kalibrierungsmittel wenigstens ein entsprechendes Ventilelement umfassen. In dieser pneumatischen Anordnung ist eine wesentliche apparative Verbesserung zu finden.

Einem bisherigen Aufbau einer gattungsgemäßen Vorrichtung ist wenigstens ein zusätzliches Ventilelement hinzugefügt oder es wird einem bereits vorhandenen Ventilelement eine entsprechend neue Funktion zugewiesen, so dass es möglich ist, bei Bedarf das Beatmungssystem zu be- oder entlüften.

Eine bevorzugte Ausführungsvariante sieht vor, dass das Ventilelement elektronisch steuerbar ist. Hierdurch kann es bedarfsweise schnell und einfach mithilfe einer entsprechend ausgestalteten Elektronikeinrichtung der vorliegenden Vorrichtung angesteuert werden.

Eine diesbezügliche Be- oder Entlüftung wird dazu verwendet, die erzeugten Signale stets optimal an die Beatmungs-Bedürfnisse des Patienten angepasst sind.

Insofern sieht eine vorteilhafte Verfahrensvariante vor, dass das Beatmungssystem belüftet oder entlüftet wird, wenn eine Kalibrierung, insbesondere eine Kalibrierung durch Bilden des (insbesondere aber nicht ausschließlich) arithmetischen Mittelwertes, rechnerisch ausgeschlossen ist.

Sollte es mit arithmetischen Mitteln nicht möglich sein, das erzeugte Signal durch rechnerische Korrektur auf seinen Ursprungswert zurück zu führen, so wird das wenigstens eine Ventilelement zur Belüftung und/oder Entlüftung betätigt, so dass in der Folge das Beatmungssystem kalibrierbar wird.

Eine Betätigung erfolgt bevorzugt, sobald ein vorbestimmbarer Schwellwert über- und/oder unterschritten wird.

Die Regelung der Betätigung des wenigstens einen Ventilelements erfolgt bevorzugt durch eine entsprechend ausgerüstete Elektronikeinrichtung der vorliegenden Vorrichtung.

Insofern ist es vorteilhaft, wenn die Vorrichtung wenigstens eine Elektronikeinrichtung umfasst, welche mit dem wenigstens einen Sensorelement zumindest zeitweise derart in Verbindung steht, dass eine Signalübertragung bzw. ein Signalaustausch zwischen dem wenigstens einen Sensorelement und der Elektronikeinrichtung ermöglicht ist.

So kann das wenigstens eine Sensorelement und die Elektronikeinrichtung beispielsweise mittels eines kabelartigen Verbindungselements miteinander in Verbindung stehen, über welches zumindest zeitweise wenigstens ein Signalaustausch erfolgt.

Idealerweise ist vorbestimmbar, in welchen zeitlichen Abständen ein Signalaustausch erfolgt. Hierdurch besteht eine weitere Möglichkeit, die Kalibrierungsgenauigkeit individuell einzustellen, falls dies erforderlich oder gewünscht ist.

Verfahrenstechnisch ist es weiter vorteilhaft, wenn das wenigstens eine Sensorelement die erzeugten Signale einzeln oder als vorbestimmbare Signalpakete an die wenigstens eine Elektronikeinrichtung übermittelt.

Darüber ist auch denkbar, dass die wenigstens eine Elektronikeinrichtung die zeugten Signale einzeln oder als vorbestimmbare Signalpakete von dem wenigsten einen Sensorelement abruft.

Vorteilhaft ist es auch, wenn die Vorrichtung und insbesondere die Elektronikeinrichtung weiterhin wenigstens eine Steuereinrichtung umfasst, welche bevorzugt die von dem wenigstens einen Sensorelement erzeugten Signale erfasst und weiterverarbeitet.

Bevorzugt kann insbesondere auch die Elektronikeinrichtung weiterhin eine Regeleinrichtung umfassen, mittels welcher ein Gasstrom zur Beatmung des Patienten regulierbar und/oder anpassbar ist.

Bevorzugt regelt die Regeleinrichtung wenigstens eine Ventilationseinrichtung des Beatmungssystems.

Bevorzugt umfasst die Vorrichtung und insbesondere die Elektronikeinrichtung weiterhin wenigstens eine Displayeinrichtung zur Visualisierung der durch das wenigstens eine mit dem Patienten wechselwirkende Sensorelements erzeugten Signale.

Gegenüber dem Stand der Technik wird der Anwender durch die Möglichkeit die erzeugten Signale am Monitor zu verfolgen in die Lage versetzt, optisch schnell zu erkennen, wie sich die erzeugten Signale, welche zur zeitlichen Steuerung der Beatmung verwendet werden, verhält.

Der Anwender kann anhand der graphischen Darstellung der erzeugten Signale besonders einfach eine Empfindlichkeit bzw. eine entsprechende Triggerschwelle des Beatmungssystems einstellen, ab welcher das Beatmungssystem eine Eigenatmung des Patienten erkennt.

Vorteilhafterweise kann hierdurch eine sofortige Anpassung des Beatmungssystems an die Respirator-Patient-Situation ermöglicht werden, da der Anwender bereits an der Displayeinrichtung etwa eines Ventilators des Beatmungssystems die Auswirkung der Verstellung der Empfindlichkeit vorhersehen kann.

Idealerweise wird das erzeugte Signal als Näherung einer Volumenkurve visualisiert. Die Darstellung der erzeugten Signale an einer Displayeinrichtung eines diesbezüglichen Ventilators ist hierbei vorzugsweise derart gewählt, dass sie einer Volumenkurve zumindest nahe kommt. Dies ist vorliegend besonders vorteilhaft, da die Volumenkurve in etwa dem Vorgang der physiologischen/physikalischen Signalentstehung entspricht.

Die Displayeinrichtung ist bevorzugt als Monitor oder als Touch-Screen ausgebildet.

Die mittels der Displayeinrichtung darstellbaren Signale können hierbei als Rohsignale, also wie von dem wenigstens einen Sensorelement erzeugt, dargestellt sein.

Ferner ist es aber auch denkbar, dass insbesondere mittels der Elektronikeinrichtung zumindest teilweise eine Signalbearbeitung erfolgt, um die vom wenigstens einen Sensorelement erzeugten Signale graphisch darzustellen.

Weiterhin umfasst die Vorrichtung und insbesondere die Elektronikeinrichtung eine Speichereinheit, mittels welcher die Signaldaten speicherbar sind. Somit sind auch Übersichtsdarstellungen über bereits vergangene Zeitintervalle darstellbar.

Gegenüber dem Stand der Technik, so wie er beispielsweise in einigen herkömmlichen Respiratoren zu finden ist, kann das erfindungsgemäße Verfahren dahingehend weiter verbessert werden, wenn nicht nur der Beginn einer Atembemühung erfasst wird, sondern darüber hinaus noch die Möglichkeit besteht, das Ende der Inspiration zu erfassen.

Damit wird es erstmals möglich, mit einfachsten Mitteln eine vollständige zeitliche Synchronisation zwischen einem Ventilator und der Atemtätigkeit des Patienten zu erreichen, ohne das am Beatmungssystem bzw. an einem entsprechenden Respirator spezielle Einstellungen vorgenommen werden müssen.

Insofern ist es vorteilhaft, wenn sowohl ein Beginn als auch ein Ende einer Inspiration ermittelt wird.

Vorteilhafterweise umfasst die erfindungsgemäße Vorrichtung wenigstens ein Sensorelement, welches bevorzugt als Respirations-Sensorelement ausgebildet ist. Als Respirations-Sensorelement ist hierbei auch ein Sensorelement zu verstehen, welches beispielsweise die Atemgeschwindigkeit und/oder das Atemvolumen erfasst.

Je nach Anwendung können selbstverständlich auch mehr als ein Sensorelement vorgesehen sein, wie beispielsweise ein bis fünf derartiger Sensorelemente.

Vorteilhafterweise bietet die Vorrichtung eine neue Alternative gegenüber herkömmlichen mit einem Flowsensorelement bzw. einem Drucksensorelement ausgerüsteten Beatmungssystemen aus dem Stand der Technik.

Jedenfalls kann das vorliegende wenigstens eine Sensorelement speziell als Respirations-Sensorelement unkomplizierter an dem Patienten angeordnet werden.

Vorteilhaft erfolgt eine lösbare Befestigung des wenigstens einen Sensorelements vorzugsweise im Abdomenbereich des Patienten.

Insofern ist es vorteilhaft, wenn das wenigstens eine Sensorelement ein Respirations-Sensorelement umfasst, welches im Abdomenbereich des Patienten anordenbar ist.

Die Befestigung kann hierbei mittels wenigstens eines zusätzlichen Klebeelements, beispielsweise mittels eines medizinischen Klebebands, erfolgen.

Eine konstruktiv noch einfachere Anbringung an dem Patienten kann erfolgen, wenn das wenigstens eine Sensorelement einen Abschnitt umfasst, mittels welchem das wenigstens eine Sensorelement am Abdomenbereich des Patienten auf der Haut lösbar fixierbar ist.

Hierzu kann beispielsweise ein hautverträgliches Klebemittel vorgesehen sein, welches zumindest teilweise auf dem Abschnitt aufgebracht ist.

Dieser Abschnitt ist hierbei idealerweise variabel ausgebildet und kann beispielsweise rund oder auch polygonal ausgebildet sein.

Darüber hinaus ist es vorteilhaft, wenn die erfindungsgemäße Vorrichtung wenigstens eine Datenschnittstelle zur Datenübertragung zwischen dem wenigstens einen Sensorelement und insbesondere der Elektronikeinrichtung umfasst.

Im einfachsten Ausführungsbeispiel ist die Schnittstelle als Steckverbindung ausgebildet.

Darüber hinaus ist es möglich, dass eine Signalübertragung beispielsweise mittels eines Kabelelements, eines Glasfaserelements oder kabelfrei, wie etwa durch Funkwellen, Radiowellen, mittels eines RFID-Chips oder dergleichen, vorgenommen werden kann. Bei den kabelfreien Alternativen sind wenigstens eine entsprechende Signalsendeeinheit und wenigstens eine Signalempfangseinheit bereitzustellen.

An dieser Stelle sei noch angemerkt, dass die Vorrichtung auch mehrere Elektronikeinrichtungen umfassen kann, um eine schelle und betriebssichere Kalibrierung stets gewährleisten zu können.

Selbstverständlich ist die vorliegende Erfindung nicht auf die genannten Ausführungsbeispiele beschränkt, sondern anwendungsbezogen erweiterbar.

Weitere Vorteile, Ziele und Eigenschaften vorliegender Erfindung werden anhand anliegender Zeichnung und nachfolgender Beschreibung erläutert, in welchen beispielhaft einzelne Komponenten einer erfindungsgemäßen Vorrichtung zur Atmungserkennung und/oder zur Atmungsunterstützung eines Patienten dargestellt und beschrieben sind. In der Zeichnung zeigen:
- Figur 1: schematisch eine Ansicht eines Sensorelements der erfindungsgemäßen Vorrichtung;
- Figur 2: schematisch eine Ansicht einer beispielhafte Anordnung des Sensorelements aus der Figur 1 an einem Patienten;
- Figur 3: schematisch eine Ansicht einer erfindungsgemäßen Elektronikeinrichtung der erfindungsgemäßen Vorrichtung;
- Figur 4: schematisch eine Ansicht eine erfindungsgemäße Schnittstelle der erfindungsgemäßen Vorrichtung;
- Figur 5A: schematisch eine Ansicht exemplarische Bedienung der Elektronikeinrichtung aus der Figur 3; und
- Figur 5B: schematisch eine Ansicht zur Auswahl einer Triggerkurve.

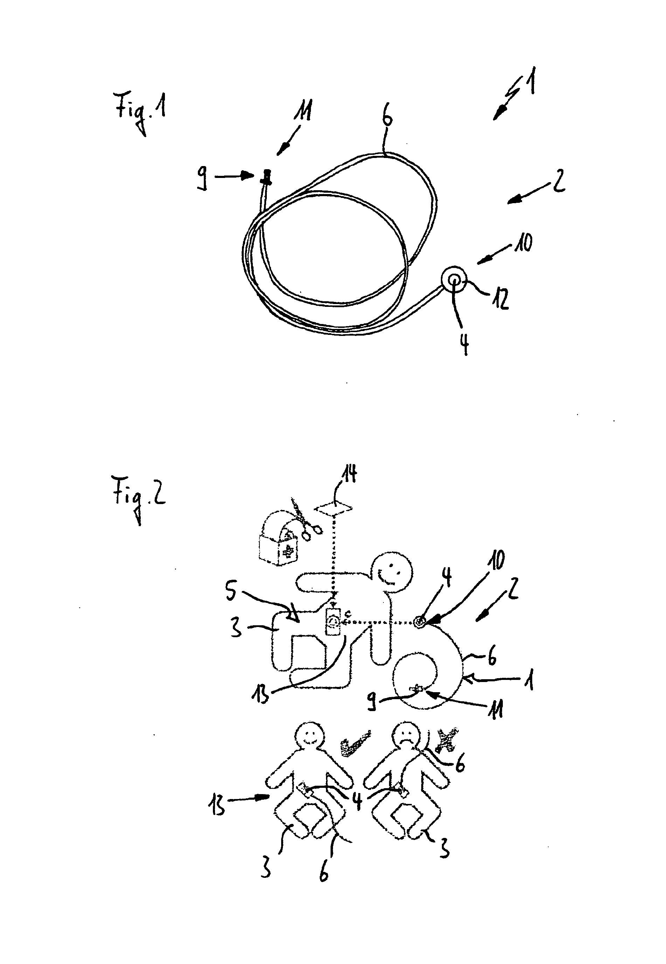

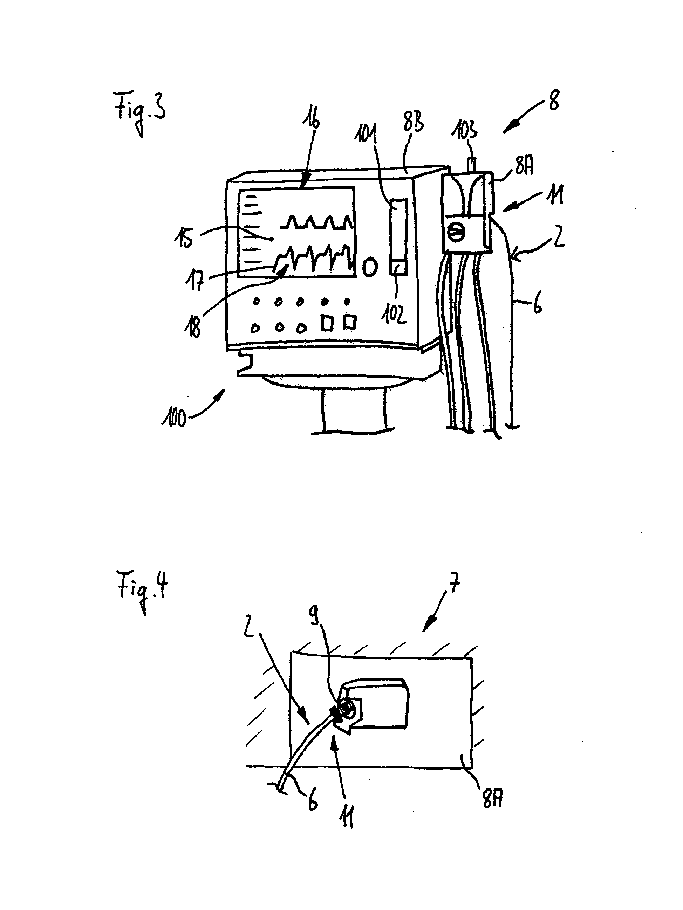

Bei dem in der Figur 1 gezeigten Sensorelement 1, handelt es sich um ein Respirations-Sensorelement 2, welches ein wesentliches Bestandteil eines Beatmungssystems 100 (siehe Figur 3) einer hier nicht näher gezeigten erfindungsgemäßen Vorrichtung zur Atmungserkennung und/oder zur Atmungsunterstützung eines Patienten 3 (siehe Figur 2) ist.

Das Sensorelement 1 besteht in diesem Ausführungsbeispiel aus einem Sensorteil 4, welches direkt auf die Haut 5 (siehe Figur 2) des Patienten 3 appliziert wird, und aus einem Kabelteil 6, mittels welchem von dem Sensorteil 4 erzeugte Signale (hier nicht explizit gezeigt) an eine Datenschnittstelle 7 (siehe Figur 4) einer Elektronikeinrichtung 8 (siehe Figur 3) der Vorrichtung übertragen werden können. Hierzu verfügt das Sensorelement 1 noch über ein elektrisches Steckverbindungsteil 9, welches mit der komplementär ausgestalteten Datenschnittstelle 7 verbunden werden kann. Die Datenschnittstelle 7 befindet sich an einer Rückseite eines Gehäuses 8A eines die Elektronikeinrichtung 8 umfassenden Respirators 8B, welcher sogleich Kalibrierungsmittel 101 zur Kalibrierung des mit dem Patienten 3 wechselwirkenden Sensorelements 1 umfasst. Die Kalibrierungsmittel 101 umfassen eine Einrichtung 102 zum Betätigen eines Ventilelements 103 zur Belüftung und/oder Entlüftung des Beatmungssystems 100.

Das Sensorteil 4 gestaltet ein erstes Ende 10 des Sensorelements 1 und das Steckverbindungsteil 9 ein zweites Ende 11 des Sensorelements 1 aus, und durch das Kabelteil 6 sind das Sensorteil 4 und das Steckverbindungsteil 9 dauerhaft miteinander verbunden.

Das Sensorteil 4 ist umfangsseitig von einem Abschnitt 12 umgeben, mittels welchem das Sensorelement 1 an der Haut 5 des Patienten 3, und zwar im Abdomenbereich 13 des Patienten 3, lösbar fixierbar ist.

Bevorzugt ist der Abschnitt 12 zumindest teilweise flexibel und/oder dehnbar ausgebildet, so dass das Sensorteil 4 bei einer Atembewegung im Abdomenbereich 13 sicher und für den Patienten 3 angenehm auf der Haut 5 befestigt verbleibt.

Gemäß der Darstellungen nach der Figur 2 ist gezeigt, wie das Sensorelement 1 korrekt und ungünstig an dem Patienten 3 befestigt ist.

Bevorzugt befindet sich der Patient 3 für eine optimale Platzierung des Sensorelements 1 in Rückenlage. Das Sensorteil 4 wird bevorzugt im Abdomenbereich13 des Patienten 3 platziert und zusätzlich noch oder je nach Ausgestaltung des Sensorteils 4 ausschließlich mit medizinischem Klebeband 14 befestigt.

Vorteilhaft steht das medizinische Klebeband 14 auf beiden Seiten des Sensorelements 1 mindestens 1 cm, bevorzugt mindestens 3 cm über, so dass das Sensorteil 4 auch bei ungünstigen Körperbewegungen des Patienten 3 verlässliche Signale ermitteln und erzeugen kann.

Ferner ist zu prüfen, ob die bezüglich des Patienten erzeugten Signale klar und deutlich an einem Monitor 15 einer Displayeinrichtung 16 der Elektronikeinrichtung 8 angezeigt werden.

Die optische Darstellung der erzeugten Signale erfolgt bevorzugt in einer zweiten Kurve 17, welche als untere Kurve eine Triggerkurve 18 an dem Monitor 15 abgebildet ist. Anhand dieser Triggerkurve 18 kann insbesondere ein Schwellenwert bezüglich der Eigenatmung des Patienten 3 besonders einfach eingestellt werden.

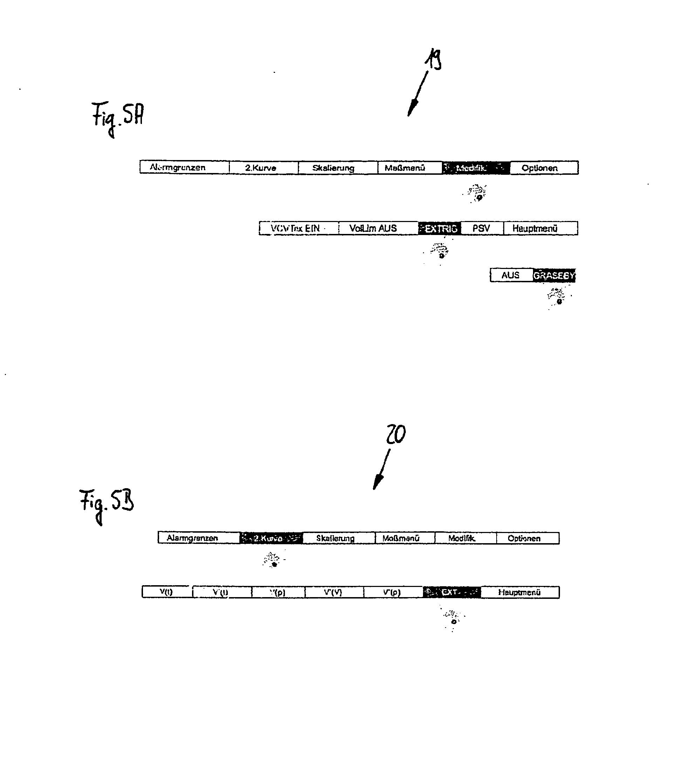

Gemäß den Darstellungen der Figuren 5A und 5B sind exemplarische Bedienungen an Bedienungsmenüs 19 und 20 der Displayeinrichtung 16 der Elektronikeinrichtung 8 dargestellt, wobei hinsichtlich der Darstellung nach Figur 5A ein Untermenü zur Aktivierung des Sensorelements 1 mit den Hauptschritten:
■ Menü "MODIFIK." der VORRICHTUNG öffnen
■ EXTRIG (externer Trigger) auswählen.
■ Es öffnet sich ein Untermenü mit den folgenden Optionen:

Aus Deaktiviert den externen Respirations-Sensor
GRASEBY Aktiviert den externen Respirations-Sensor
gezeigt ist.

Gemäß der Darstellung der Figur 5B ist die Bedienung zur Auswahl der Triggerkurve 18 schematisch dargestellt.

Um sich die Kurve des externen Triggers anzeigen lassen zu können, ist das Menü "2. KURVE" zu öffnen und die Option "EXTRIG" auszuwählen. Die externe Triggerkurve 18 wird als zweite Kurve 17 an der Displayeinrichtung 16 angezeigt.

Es versteht sich, dass es sich bei dem vorstehend erläuterten Ausführungsbeispiel lediglich um eine erste Ausgestaltung der erfindungsgemäßen Vorrichtung zur Durchführung des erfindungsgemäßen Verfahrens handelt. Insofern beschränkt sich die Ausgestaltung der Erfindung nicht auf dieses Ausführungsbeispiel.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Sensorelement
- 2: Respirations-Sensorelement
- 3: Patient
- 4: Sensorteil
- 5: Haut
- 6: Kabelteil
- 7: Datenschnittstelle
- 8: Elektronikeinrichtung
- 8A: Gehäuse
- 8B: Respirator
- 9: Steckverbindungsteil
- 10: erstes Ende
- 11: zweites Ende
- 12: Abschnitt
- 13: Abdomenbereich
- 14: medizinisches Klebeband
- 15: Monitor
- 16: Displayeinrichtung
- 17: Kurve
- 18: Triggerkurve
- 19: erstes Bedienungsmenü
- 20: zweites Bedienungsmenü
- 100: Beatmungssystem
- 101: Kalibrierungsmittel
- 102: Einrichtung zum Betätigen
- 103: Ventilelement

## Patentansprüche

1. Verfahren zur Atmungserkennung und/oder zur Atmungsunterstützung eines Patienten (3) mit einem Beatmungssystem (100) zur synchronisierten Beatmung des Patienten (3), bei welchem mittels wenigstens einem mit dem Patienten (3) wechselwirkenden Sensorelements (1, 2) eine Eigenatmung des Patienten (3) ermittelt wird, und bei welchem das Beatmungssystem (100) durch mittels des wenigstens einen mit dem Patienten (3) wechselwirkenden Sensorelements (1, 2) erzeugte Signale gesteuert wird, **dadurch gekennzeichnet, dass**
das wenigstens eine mit dem Patienten (3) wechselwirkende Sensorelement (1,2) und/oder die hierdurch erzeugten Signale während der Ermittlung der Eigenatmung kalibriert werden, indem die erzeugten Signale rechnerisch korrigiert werden.

2. Verfahren nach Anspruch 1,
**dadurch gekennzeichnet, dass**
das Beatmungssystem (100) belüftet oder entlüftet wird, wenn eine Kalibrierung rechnerisch ausgeschlossen ist.

3. Verfahren nach Anspruch 1 oder 2,
**dadurch gekennzeichnet, dass**
mittels des wenigstens einem mit dem Patienten (3) wechselwirkenden Sensorelements (1, 2) sowohl ein Beginn als auch ein Ende einer Inspiration ermittelt wird.

4. Verfahren nach wenigstens einem der vorangegangenen Ansprüche,
**dadurch gekennzeichnet, dass**
die erzeugten Signale durch Anwenden wenigstens einer mathematischen Operation und insbesondere durch Bilden wenigstens eines Mittelwertes in Bezug auf zuvor erzeugte Signale korrigiert werden.

5. Vorrichtung zur Atmungserkennung und/oder zur Atmungsunterstützung eines Patienten (3) mit einem Beatmungssystem (100) zur synchronisierten Beatmung des Patienten (3), mit Mitteln zur Ermittlung einer Eigenatmung des Patienten (3) und mit Mitteln zur synchronisierten Steuerung des Beatmungssystems (100) in Abhängigkeit von einer ermittelten Eigenatmung, bei welcher die Mittel zur Ermittlung der Eigenatmung wenigstens ein mit dem Patienten (3) wechselwirkendes Sensorelement (1, 2) umfassen, insbesondere zur Durchführung des Verfahrens nach einem der vorstehenden Merkmale,
**dadurch gekennzeichnet, dass**
die Vorrichtung Kalibrierungsmittel (101) zur Kalibrierung des wenigstens einen mit dem Patienten (3) wechselwirkenden Sensorelements (1, 2) umfasst, mittels welchen das wenigstens eine mit dem Patienten (3) wechselwirkende Sensorelement (1, 2) und/oder die hierdurch erzeugten Signale während der Ermittlung der Eigenatmung kalibriert werden.

6. Vorrichtung nach Anspruch 5,
**dadurch gekennzeichnet, dass**
die Kalibrierungsmittel (101) eine Einrichtung (102) zum Betätigen eines Ventilelements (103) zur Belüftung und/oder Entlüftung des Beatmungssystems (100) umfasst.

7. Vorrichtung nach Anspruch 6,
**dadurch gekennzeichnet, dass**
das Ventilelement (103) elektronisch steuerbar ist.

8. Vorrichtung nach einem der Ansprüche 5 bis 7,
**dadurch gekennzeichnet, dass**
das wenigstens eine Sensorelement (1, 2) ein Respirations-Sensorelement (2) umfasst, welches im Abdomenbereich (13) des Patienten (3) anordenbar ist.

9. Vorrichtung nach einem der Ansprüche 5 bis 8,
**dadurch gekennzeichnet, dass**
das wenigstens eine Sensorelement (1, 2) einen Abschnitt (12) umfasst, mittels welchem das wenigstens eine Sensorelement (1, 2) am Abdomenbereich (13) des Patienten (3) auf der Haut (5) lösbar fixierbar ist.

10. Vorrichtung nach einem der Ansprüche 5 bis 9,
**dadurch gekennzeichnet, dass**
das Beatmungssystem (100) ein Respirator (8B) umfasst.

11. Vorrichtung nach einem der Ansprüche 5 bis 10,
**dadurch gekennzeichnet, dass**
die Vorrichtung eine Displayeinrichtung (16) zum visuellen Anzeigen der durch das wenigstens eine mit dem Patienten (3) wechselwirkende Sensorelements (1, 2) erzeugten Signale umfasst.
